## Europäisches Patentamt

## European Patent Office

## Office européen des brevets

(19)

(11) Veröffentlichungsnummer: **0 073 455**
**B1**

(12) **EUROPÄISCHE PATENTSCHRIFT**

(45) Veröffentlichungstag der Patentschrift:
23.04.86

(51) Int. Cl.⁴: **A 61 B 17/58**

(21) Anmeldenummer: **82107748.4**

(22) Anmeldetag: **24.08.82**

(54) Spannvorrichtung mit einer Osteosynthese-Platte.

(30) Priorität: **28.08.81 DE 3134120**

(43) Veröffentlichungstag der Anmeldung:
**09.03.83 Patentblatt 83/10**

(45) Bekanntmachung des Hinweises auf die Patenterteilung:
**23.04.86 Patentblatt 86/17**

(84) Benannte Vertragsstaaten:
**AT BE CH DE FR GB IT LI NL SE**

(56) Entgegenhaltungen:
**CH - A - 373 516**
**DE - A - 2 802 090**
**DE - B - 2 911 386**

(73) Patentinhaber: **Härle, Anton, Dr., Drechslerweg 40,
D-4400 Münster-Roxel (DE)**

(72) Erfinder: **Härle, Anton, Dr., Drechslerweg 40,
D-4400 Münster-Roxel (DE)**

(74) Vertreter: **Habbel, Hans-Georg, Dipl.-Ing.,
Postfach 3429 Am Kanonengraben 11, D-4400 Münster
(DE)**

ACTORUM AG

## Beschreibung

Die Erfindung bezieht sich auf eine Spannvorrichtung mit einer osteosynthetischen Spannplatte, die mit Schraubenlöchern und einem Langloch versehen ist, mit einem lösbar an der Spannplatte anbringbaren Kraftübertragungsglied, das einen Drehzapfen zum Einleiten einer Drehkraft aufweist und mit einem durch das Langloch hindurchführbaren, im Knochen fixierbaren und als Widerlager dienenden Stift zusammenwirkt.

In der Osteosynthese ist bekannt, Knochenteile unter Verwendung von Druckplatten zusammenzupressen. Es ist ferner bekannt, zur Druckerzeugung eine Spannvorrichtung vorzusehen, die an einem Knochenfragment (normalerweise das diaphysäre = zur Knochenmitte gerichtete) festgeschraubt und mit deren Hilfe dann die mit dem anderen Knochenfragment verschraubte Platte herangezogen wird, um die beiden Fragmente zusammenzupressen. Anschliessend wird die Platte auch mit dem zweiten Knochenfragment verschraubt und die Spannvorrichtung entfernt.

Nachteilig bei der bekannten Spannvorrichtung ist die grosse Wundlänge, welche sich dadurch ergibt, dass die Spannvorrichtung ausserhalb der Platte am Knochen fixiert wird. Da die Spannvorrichtung in der Regel mit Hilfe eines Schlüssels betätigt wird, wird dadurch die Wundlänge noch weiter vergrössert. Ein anderer Nachteil liegt in der Schwächung des Knochens, welcher ausserhalb der Platte zur Befestigung der Spannvorrichtung gebohrt wird. Um diese Knochenschwächung möglichst gering zu halten, wird deshalb die Befestigungsschraube für die Spannvorrichtung nur in eine Kortikalis eingeschraubt. Dies hat wiederum den Nachteil einer verhältnismässig geringen Haltekraft zur Folge.

Es sind ferner osteosynthetische Druckplatten bekannt geworden, bei denen eine oder mehrere Schraubenlöcher rampenartige Lochwände aufweisen. Beim Einschrauben einer Knochenschraube gleitet der Schraubenkopf auf der rampenartigen, schiefen Ebene und bewirkt eine Relativverschiebung zwischen Platte und zugeordnetem Knochenfragment (DE-PS 1 566 153). Bei derartigen Druckplatten entfällt zwar eine besondere Spannvorrichtung, andererseits ist die Kompression in starkem Masse abhängig von der Auszugsfestigkeit der Schraube. Dadurch dass die Bewegung der Schraube und der Platte senkrecht zueinander verlaufen, wird nur ein verhältnismässig geringer Teil der Schraubenkraft in die Verschiebung der Druckplatte umgesetzt. Es kommt dementsprechend zu einer starken Zugbelastung der Knochenschraube. Wegen der ungünstigen Kraftübertragung zwischen Schraube und Druckplatte ist das Aufbringen der Kompressionskraft an den Frakturflächen relativ unkontrolliert.

Ferner ist der auf diese Weise erzielbare Verschiebeweg verhältnismässig gering. Ist eine Knochenschraube völlig eingeschraubt, steht kein weiterer Verschiebeweg mehr zur Verfügung. Bei einer Lockerung der Schraube kommt es zudem wieder zu einer Aufhebung des Kompressionsdruckes. Als weiteres ungünstiges Faktum ist festzustellen, dass es beim Spannvorgang naturgemäss zu einer zunehmenden Reibung auch zwischen Platte und Knochen auf der Kompressionsseite kommt, die einer weiteren Relativverschiebung entgegensteht. Auch die wegen lokaler Nekrosen (druckschädigungsbedingt) ablaufende, physiologische Gewebsauflösung von Knochenhaut und Knochenvorsprüngen lässt eine postoperative Annäherung von Platte und Knochen zu und führt zu einer Verringerung/Aufhebung des Pressdruckes.

Den Nachteil der oben angeführten, ausserhalb der Druckplatte angebrachten Spannvorrichtung beseitigt eine weitere bekannte Spannvorrichtung, welche auf die Druckplatte aufgesetzt wird. Die Druckplatte besitzt an einem Ende einen gezahnten Schlitz, der auf der Kompressionsseite des Knochens angeordnet und über den ein Loch in den Knochen gebohrt wird, das einen Widerlagerstift aufnimmt, welcher in die Spannvorrichtung hineinragt, welche U-förmig auf die Druckplatte im Bereich des gezahnten Schlitzes gesetzt wird. Ein innerhalb des Schlitzes angeordnetes Zahnsegment wird mit Hilfe eines von oben zum Einsatz gebrachten Drehwerkzeuges verdreht, um die Relativverschiebung zwischen Druckplatte und Knochensegment zu erzielen. Die bekannte Spannvorrichtung sieht ferner ein Feststellelement vor, um nach beendetem Kompressionsvorgang die Spannvorrichtung in ihrer Lage auf der Druckplatte zu fixieren.

Auch die zuletzt erwähnte, bekannte Spannvorrichtung ist mit einigen Nachteilen behaftet. Der gezahnte Schlitz verlängert die Platte um einen nicht unerheblichen Betrag und verhindert im übrigen, dass die Platte unterminiert gelegt werden kann. Da ferner nur eine geringe Anzahl von Zähnen im Schlitz untergebracht wird, ist eine erhebliche Drehkraft erforderlich, um den nötigen Kompressionsdruck zu erzeugen. Die grosse, vom Operateur aufzubringende Drehkraft erschwert ein kontrolliertes Arbeiten. Weiterhin kann geschehen, dass der als Widerlager dienende Stift kippt und zu einer Verformung des Loches im Knochen führt mit der Folge, dass die später einzusetzende Schraube nur mangelhaft fixiert werden kann.

Ausserdem kommt es zu einer Haftungsreibung der mit dem Widerlagerstift verbundenen Kontaktvorrichtung gegenüber der Platte, da die Rotationskräfte beim Spannvorgang eine rechtwinklig zur Plattenachse wirkende Kraft hervorrufen, die neutralisiert werden muss.

Der Erfindung liegt daher die Aufgabe zugrunde, eine Spannvorrichtung mit einer osteosynthetischen Spannplatte zu schaffen, welche die erwähnten Nachteile vermeidet, einfach aufgebaut, leicht bedienbar und operationstechnisch günstig ausgebildet ist sowie die Aufbringung eines kontrollierten Druckes auf die Knochensegmente ermöglicht.

Diese Aufgabe wird bei einer Spannvorrichtung der eingangs genannten Art dadurch gelöst, dass ein Kraftübertragungsglied aus zwei relativ zueinander in Richtung der Längsachse der Spannplatte bewegbaren Spannelementen besteht, zwischen denen eine mit dem Drehzapfen verbundene Getriebeanordnung zur Erzielung der Relativbewegung angeordnet ist, das plattenfeste Spannelement mittels einer Schraubverbindung unverschiebbar an der Spannplatte befestigt ist und das bewegliche Spannelement eine mit dem Kopf des Stiftes in Eingriff bringbare Anschlagfläche aufweist.

Die Erfindung ist mit einer Reihe von erheblichen Vorteilen verknüpft. Die erfindungsmässige Spannvorrichtung kann für eine übliche Spannplatte normaler Länge verwendet werden, welche, wie bereits erwähnt, auf der Kompressionsseite mit einem Langloch versehen ist. Die Länge des Langloches bestimmt sich nach dem theoretisch maximal auftretenden Verschiebeweg bei der Kompression. Der Unterschied zu herkömmlichen Kompressionsplatten besteht darin, dass mindestens ein Schraubenloch so auszubilden ist, dass die Spannvorrichtung verschiebefest auf der Spannplatte befestigt werden kann. Eine Ausgestaltung der Erfindung sieht hierzu vor, dass das Schraubenlauch zur Befestigung des plattenfesten Spannelements ein Gewinde aufweist. Eine weitere Ausgestaltung sieht zur verschiebefesten Aufnahme von Schraubenköpfen dienende Einbuchtungen im Langloch vor.

Wegen der üblichen Länge der Spannplatte und der Möglichkeit, die Spannvorrichtung zwischen den Enden der Platte anzubringen, ist für den Einsatz der Erfindung lediglich eine kleine Wundlänge nötig und die Platte selbst kann unterminiert gelegt werden.

Das bewegliche Spannelement der Spannvorrichtung stützt sich quer zur Stiftachse am Kopf ab (radial), so dass im Gegensatz zu bekannten Spannvorrichtungen der Stift nicht in Auszugsrichtung belastet ist. Mithin ist die Belastung des vorgebohrten Loches im Knochen verhältnismässig gering, so dass das Loch im Anschluss zur Aufnahme einer normalen Knochenschraube geeignet bleibt. Die Belastung des Stiftes bzw. des vorgebohrten Loches im Knochen als Widerlager ist auch deshalb gering, weil grosse Reibungskräfte und Kraftkomponenten in Achsrichtung des Stiftes nicht auftreten. Reibung entsteht in der Getriebeanordnung und bei der Relativbewegung der Spannelemente. Beide Reibungsfaktoren können verhältnismässig klein gehalten werden. Dies trägt dazu bei, dass die vom Operateur aufzubringende Verstellkraft klein gehalten werden kann. Dies beruht jedoch in erster Linie auf der verwendeten Getriebeanordnung, welche eine gewünschte Übersetzung zwischen Drehzapfen, an dem die Kraft eingeleitet wird, und dem Verstellweg pro Zapfenumdrehung relativ zwischen den Spannelementen gestattet. Die geringen Reibungskräfte und die verhältnismässig geringe vom Operateur aufzubringende Drehkraft gestattet ein kontrolliertes Arbeiten und Einstellen der gewünschten Flächenpressung an den Frakturenden. Im Gegensatz zu herkömmlichen Spannvorrichtungen gestattet eine Verstellung mit Hilfe eines Drehmomentschraubenziehers eine verhältnismässig genaue Aussage über den Druck an den Frakturflächen.

Ein weiterer Vorteil der Erfindung liegt darin, dass die Spannvorrichtung auf einfache Weise auf die Spannplatte aufgebracht und von dieser entfernt werden kann. Auch die Betätigung der Spannvorrichtung unter Operationsbedingungen erweist sich als den Umständen entsprechend bequem. Die Anordnung der Spannvorrichtung kann wahlweise auf der Fixations- oder Kompressionsseite der Osteosyntheseplatte erfolgen, je nach den intraoperativen Bedingungen. Eine Ummontage ist mit wenigen Handgriffen und ohne wesentliche Zeiterfordernis zu bewerkstelligen.

Die Erfindung lässt sich nicht nur zur Kompression einer Fraktur verwenden, sondern auch zum Zwecke der Reposition. Bekanntlich müssen die Knochenfragmente zunächst in die richtige Lage zueinander positioniert werden, bevor eine Kompression erfolgen kann. Das Reponieren erfordert wegen vorhandener Muskelspannungen erhebliche Kräfte, welche mit Hilfe der erfindungsmässigen Spannvorrichtung überwunden werden können. Wird die erfindungsmässige Spannvorrichtung sowohl zum Reponieren als auch zum Komprimieren eingesetzt, wird der Widerlagerstift zweckmässigerweise in der Mitte des Langloches angeordnet, damit eine Verschiebung der Platte relativ zum Knochensegment in beiden Richtungen erfolgen kann. Schliesslich lässt sich aus den erwähnten Gründen die erfindungsmässige Spannvorrichtung auch für Operationen einsetzen, bei denen Knochen verlängert werden.

Um eine möglichst mittige Anordnung der Spannvorrichtung auf der Spannplatte zu erhalten, sehen weitere Ausgestaltungen der Erfindung vor, das Gewindeloch nur auf der Fixations- oder nur auf der Kompressionsseite, oder aber auf beiden Seiten anzuordnen.

Eine weitere Ausgestaltung der Erfindung sieht vor, dass der Widerlagerstift eine Knochenschraube ist. Dies verbindet die Vorteile einer festeren Fixation des Widerlagers im Knochen mit dem Umstand, dass diese Widerlagerschraube am Ende des Spannvorgangs nicht ausgewechselt werden muss. Zum Zwecke der Kompression wird die Knochenschraube zweckmässigerweise durch beide Kortikalisrinden eingedreht, aber nicht festgezogen. Sie ragt so weit genug aus dem Knochen heraus, damit ihr Kopf eine Angriffsfläche für das bewegliche Spannelement bietet und dieser im Langloch gleiten kann. Nach Beendigung des Kompressionsvorganges kann dann die als Widerlager dienende Knochenschraube völlig in den Knochen eingeschraubt und festgezogen werden. In diesem Zusammenhang sieht eine weitere Ausgestaltung der Erfindung vor, dass das Langloch in Längsrichtung beabstandet paarweise, gegenüberliegende Ausbuchtungen zur Aufnahme von Schraubenköpfen aufweist. Dadurch wird eine Relativverschiebung zwischen festangezogener Knochenschraube und Spannplatte verhindert.

Für die Übertragung der am Zapfen eingeleiteten Drehkraft auf das bewegliche Spannelement sind verschiedene konstruktive Möglichkeiten denkbar. Eine Ausgestaltung der Erfindung sieht hierzu vor, dass der Drehzapfen am plattenfesten Spannelement angebracht ist und mit einem Zahnradgetriebe verbunden ist, dessen Antriebselement auf das bewegliche Spannelement wirkt. Zur günstigen, hochübersetzten Kraftübertragung sieht eine weitere Ausgestaltung der Erfindung vor, dass das Antriebselement auf ein mit dem beweglichen Spannelement zusammenwirkendes Schraubspindelgetriebe wirkt.

Die Getriebeanordnung ihrerseits ist vorzugsweise ein Kegelgetriebe. Bei Verwendung eines Drehspindelgetriebes ist die Drehspindel gemäss einer weiteren Ausgestaltung der Erfindung drehbar im plattenfesten Spannelement gelagert, ein Abschnitt des beweglichen Spannelementes enthält eine mit der Spindel zusammenwirkende Gewindebohrung und das bewegliche Spannelement ist drehfest aber verschiebbar am plattenfesten Spannelement gelagert. Nach einer weiteren Ausgestaltung der Erfindung ist das bewegliche Spannelement in einer Bohrung des plattenfesten Spannelementes drehfest gelagert.

Mit der erfindungsmässigen Spannvorrichtung ist auch die Gelegenheit gegeben, die auf die Frakturflächen wirkende Kompressionskraft unmittelbar zu messen. In diesem Zusammenhang sieht eine weitere Ausgestaltung der Erfindung vor, dass eine Kraftmesseinrichtung im Kraftübertragungsweg zwischen Drehzapfen und bewegbarem Spannelement geschaltet ist. Die Kraftmesseinrichtung kann beispielsweise eine Federanordnung enthalten, wobei der Federweg ein Mass ist für den Grad der Kompression bzw. der Pressung zwischen der Frakturflächen.

In einer Alternative kann nach einer weiteren Ausgestaltung der Erfindung vorgesehen sein, dass im Kraftübertragungsweg ein fluides, inkompressibles Medium angeordnet ist und die das fluide Medium enthaltende Kammer oder Leitung mit einer Druckmessvorrichtung verbindbar ist. Der Druck des fluiden Mediums, der mit einer geeigneten Druckmessvorrichtung gemessen werden kann, ist unmittelbar ein Mass für die auf die Frakturflächen einwirkende Kraft.

Ein Ausführungsbeispiel der Erfindung wird nachfolgend anhand von Zeichnungen näher beschrieben.

Fig. 1 zeigt einen Schnitt durch eine Spannvorrichtung nach der Erfindung in Verbindung mit einer an einem Knochen liegenden Spannplatte.

Fig. 2 zeigt eine Draufsicht auf die Spannplatte nach Fig. 1.

Fig. 3 zeigt eine andere Ausführungsform für eine Spannvorrichtung nach der Erfindung.

Bevor auf die in den Zeichnungen dargestellten Einzelheiten näher eingegangen wird, sei vorangestellt, dass jedes Merkmal für sich oder in Verbindung mit Merkmalen der Ansprüche von erfindungswesentlicher Bedeutung ist.

Die Zeichnungen sind lediglich schemenhaft und nicht massstäblich.

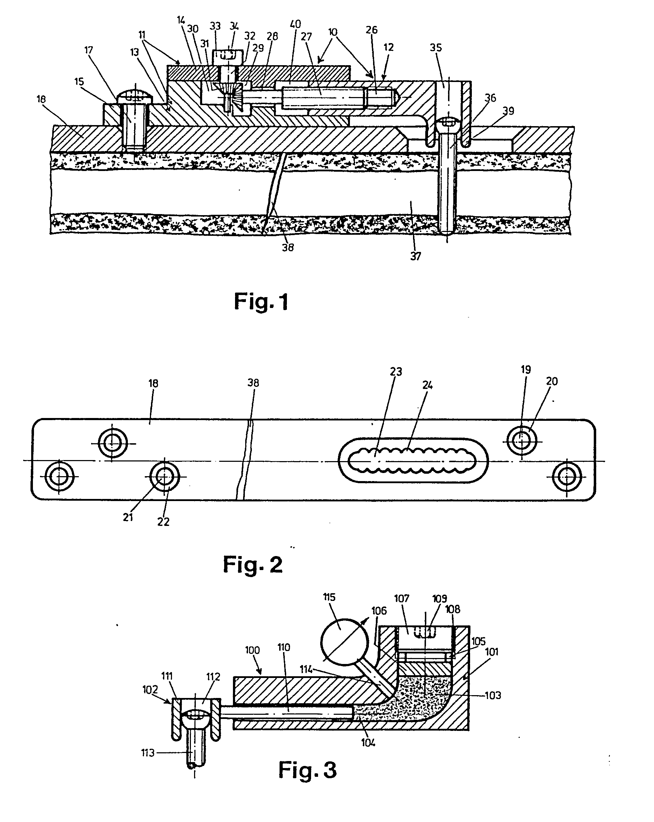

Die in Fig. 1 dargestellte Spannvorrichtung ist allgemein mit 10 bezeichnet und setzt sich aus einem plattenfesten Spannelement 11 und einem beweglichen Spannelement 12 zusammen. Die Spannelemente 11 und 12 sind plattenförmig ausgebildet. Das plattenfeste Spannelement 11 ist gehäuseartig aufgebaut und setzt sich aus einem Grundkörper 13 und einer Deckplatte 14 zusammen, wobei letztere mit Hilfe von Schrauben oder anderer geeigneter Befestigungsmittel lösbar am Grundkörper 13 befestigbar ist.

Der Grundkörper 13 besitzt an einer Stirnseite einen Ansatz 15 mit einer Öffnung zur Aufnahme einer Befestigungsschraube 17. Mit Hilfe der Befestigungsschraube 17 kann das plattenfeste Spannelement 11 lösbar an einer osteosynthetischen Spannplatte 18 befestigt werden. Die Spannplatte 18 ist in Fig. 2 in Draufsicht dargestellt. Sie ist in der üblichen Weise ausgebildet und bemessen. Sie weist einige Befestigungslöcher 19 auf mit Ansenkungen 20. Eines oder zwei dieser Löcher 19/21 mit einer Ansenkung 20/22 ist/sind mit Gewindebohrung ausgestattet. Sie nimmt die Befestigungsschraube 17 auf, um das plattenfeste Spannelement 11 unverschiebbar und lösbar an der Spannplatte 18 zu befestigen. Hierbei liegt, wie aus Fig. 1 zu erkennen, die flache Unterseite des Körpers 13 auf der Spannplatte 18 auf. Die Spannplatte weist zwischen den Enden ein Langloch 23 auf mit in Längsrichtung beabstandeten, sich paarweise gegenüber liegenden Einbuchtungen 24.

Wie aus Fig. 1 ferner zu erkennen ist, bilden Deckplatte 14 und Körper 13 eine Ausnehmung 40, welche im Querschnitt quadratisch oder rechteckig ist zur Aufnahme des passend und verschiebbar darin geführten, beweglichen Spannelementes 12. Wahlweise kann der Querschnitt der Ausnehmung 40 so ausgebildet sein, dass andere, mit Kantenflächen ausgestattete Zylinderformen von 3- bis 9eckig oder rund mit Nuten am Element 40 oder 12 zum Einsatz kommen. Das Spannelement 12 weist auf der in das Innere der Ausnehmung 40 weisenden Seite eine Gewindesackbohrung 26 auf, die eine Gewindespindel 27 aufnimmt, welche über eine zwischen Deckplatte 14 und Körper 13 gebildetes Lager 28 mit einem Kegelrad 29 verbunden ist. Das Kegelrad 29 ist in einer von der Deckplatte 14 und dem Körper 13 gebildeten Kammer 30 angeordnet und kämmt mit einem Kegelrad 31, dessen Achse senkrecht zur Achse des Kegelrades 29 ist und auf einer Seite im Körper 13 und auf der anderen Seite in einer Öffnung der Deckplatte 14 drehbar gelagert ist. Das Kegelrad 31 ist über einen durch die Öffnung in der Deckplatte 14 hindurchgeführten Wellenabschnitt 32 mit einem Zapfen 33 verbunden, der einen nach aussen weisenden Innensechskant 34 besitzt.

Das bewegliche Spannelement 12 besitzt am freien Ende eine Öffnung 35, mit deren Hilfe der Kopf der Widerlagerschraube 16 erfasst werden kann, welche durch das Langloch 23 in einen Knochen 37 eingeschraubt ist. Die die Öffnung 35 begrenzenden Wände sind mit dem Langloch kongruenten Zapfen 39 ausgestattet, um so eine rotationssichere Fixation des Spannelementes auf der Spannplatte zu sichern und die Widerlagerfläche für die Schraube 36 zu vergrössern. Der Knochen 37 hat eine Fraktur an der Stelle 38. Die Arbeitsweise der gezeigten Spannvorrichtung ist wie folgt:

Zunächst wird die osteosynthetische Druckplatte 18 mit Hilfe geeigneter Knochenschrauben verbunden – in Fig. 1 links der Fraktur 38. Anschliessend wird die Spannvorrichtung 10 mit Hilfe der Befestigungsschraube 17, welche im Gewindeloch 21 eingeschraubt ist, unverschiebbar befestigt. Vorzugsweise wird die Osteosyntheseplatte mit schon von der assistierenden Schwester befestigten Spannvorrichtung angereicht, was zu einer Zeitersparnis und zu einer Verringerung der in den Operationswunde erforderlichen Handgriffe führt.

Der Plattenfixationsvorgang wird durch die montierte Spannvorrichtung nicht behindert. Zur Erzielung eines maximalen Kompressionshubes wird durch entsprechende Drehung am Zapfen 33 mit Hilfe eines Schraubenziehers, wie er auch zum Eindrehen der Knochenschrauben verwendet wird, das bewegliche Spannelement 12 nach rechts (in Fig. 1) ausgefahren und eine am rechten Ende des Langloches 23 durch die Öffnung 35 in den Knochen 37 eingeschraubte Schraube 36 mit ihrem etwas herausragenden Kopf gefasst (Fig. 1 zeigt bereits den beendeten Kompressionsvorgang). Anschliessend wird durch entgegengesetzte Drehung am Zapfen 34 das Spannelement 12 in die Ausnehmung 40 hineingezogen, so dass über die Widerlagerschraube 36 das rechte Knochenfragment gegen das linke angepresst wird. Dieser Anpressvorgang erfolgt bis zum Erreichen einer vom Operateur vorgegebenen Anpresskraft. Anschliessend wird auch der rechte Teil der Druckplatte 18 über Befestigungsschrauben mit dem Knochen 37 verschraubt. Schliesslich wird auch die Schraube 36, welche in Fig. 1 nicht ganz eingeschraubt dargestellt ist, durch eine weitere Umdrehung fest angezogen. Alternativ kann auch eine andere Schraube verwendet werden. Ihr Kopf gelangt in eine der Einbuchtungen 24, so dass sie ebenfalls dazu beiträgt, die erreichte Position der Knochenfragmente beizubehalten. Die Spannvorrichtung ist dann durch Lösen der Befestigungsschraube 17 mit einem Handgriff entfernbar und es kann erforderlichenfalls eine weitere Schraube im Langloch zur Plattenfixation eingesetzt werden, die dann ebenfalls in einer Einbuchtung 24 einen festen Sitz erhält.

Die in Fig. 1 dargestellte Spannvorrichtung kann auch zum Reponieren verwendet werden. Für diesen Fall wird die Schraube 36 annähernd in der Mitte des Langlochs 23 in den Knochen 37 geschraubt. Durch Drehung des Zapfens 33 mit Hilfe eines Schraubenziehers wird das bewegliche Spannelement 12 gegenüber dem plattenfesten Spannelement 11 nach aussen (in Fig. 1 nach rechts) gedrückt, um die beiden Knochenfragmente so weit auseinanderzubewegen, dass sie in die richtige Lage zueinander gebracht werden können, was nach ausreichender Distraktion durch Anziehen der Schraube 36 erreicht wird. Anschliessend erfolgt der Kompressionsvorgang in umgekehrter Drehrichtung in der oben beschriebenen Art.

Der zuletzt erwähnte Vorgang ist im übrigen auch zum Auseinanderbewegen von Knochenfragmenten geeignet bei Knochenverlängerungsoperationen.

Dieser oben beschriebene Vorgang zur Frakturreposition und anschliessender Kompression kann auch bei Fixation der Spannvorrichtung 10 im Loch 19 erfolgen, nur sind dann gegensinnige Drehbewegungen am Zapfen 33 auszuführen. In besonderen Einzelfällen kann die Spannvorrichtung leicht auch während der Anwendung, z.B. nach erreichter Reposition, die dann durch die Schraube 36 gesichert ist, vom Loch 21 nach Loch 19 ummontiert werden zur Erzielung einer Kompressionswirkung für die Ausführungsform nach Fig. 3.

Wie aus Fig. 1 ersichtlich, ist die gezeigte Spannvorrichtung verhältnismässig kompakt und in der Höhe nur geringfügig grösser als die Druckplatte 18. Sie kann im übrigen sehr leicht sterilisiert werden, indem durch Entfernen des Deckels 14 alle Flächen und Hohlräume freigegeben werden.

Fig. 3 zeigt äusserst schematisch eine Spannvorrichtung 100 mit einem plattenfesten Spannelement 101 und einem beweglichen Spannelement 102. Das plattenfeste Spannelement 101 besitzt eine innere Kammer 103, welche am linken Ende des Spannelementes 101 über eine Zylinderbohrung 104 und am oberen Ende über eine Zylinderbohrung 105 Verbindung nach aussen hat. In letzterer ist gleitend bewegbar ein Kolben 106 angeordnet, über dem ein ein Aussengewinde aufweisender Stopfen angeordnet ist, der in einem Innengewinde 108 der Bohrung 105 drehbar angeordnet ist. Der Stopfen 107 besitzt einen Innensechskant 109. Das Spannelement 102 besitzt einen Kolbenabschnitt 110, der gleitend und dichtend in der Bohrung 104 angeordnet ist. Der aussen liegende Abschnitt 111 ist mit einer Öffnung 112 versehen zur Aufnahme des Kopfes einer Schraube 113, ähnlich der Widerlagerschraube 36 nach Fig. 1. Die Kammer 103 ist mit einem fluiden, inkompressiblen Medium gefüllt, dass im übrigen sich gegenüber Körperflüssigkeiten neutral verhält. Durch Drehung des Stopfens 107 kann der Kolben 105 nach unten gedrückt werden, wodurch der Kolben 110 nach aussen wandert und auf die Schraube 113 einen Schub in Richtung nach links (nach Fig. 3) ausübt.

Die Kammer 103 ist über eine Bohrung 114 mit einem Druckmessgerät 115 verbunden. Das Druckmessgerät misst den Druck in der Kammer 103, welcher direkt proportional ist der auf die Schraube 113 ausgeübten Schubkraft.

Es versteht sich, dass mit der in Fig. 3 gezeigten Spannvorrichtung lediglich eine Relativbewegung der Spannelemente 101 und 102 voneinander fort erreicht werden kann.

**Patentansprüche**

1. Spannvorrichtung mit einer osteosynthetischen Spannplatte, die mit Schraubenlöchern und einem Langloch versehen ist, sowie mit einem lösbar an der Spannplatte anbringbaren Kraftübertragungsglied, das einen Drehzapfen zum Einleiten einer Drehkraft aufweist und mit einem durch das Langloch hindurchführbaren, im Knochen fixierbaren und als Widerlager dienenden Stift zusammenwirkt, dadurch gekennzeichnet, dass das Kraftübertragungsglied (10) aus zwei relativ zueinander in Richtung der Längsachse der Spannplatte (18) bewegbaren Spannelementen (11, 12) besteht, zwischen denen eine mit dem Drehzapfen (33) verbundene Getriebeanordnung zur Erzeugung der Relativbewegung angeordnet ist, dass das plattenfeste Spannelement (11) mittels einer Schraubverbindung unverschiebbar an der Spannplatte (18) befestigbar ist und dass das bewegliche Spannelement (12) eine mit dem Kopf des Stiftes (36) in Eingriff bringbare Anschlagfläche aufweist.

2. Spannvorrichtung nach Anspruch 1, dadurch gekennzeichnet, dass die Schraubverbindung zur Befestigung des plattenfesten Spannelementes (11) ein Schraubenloch (21) mit Gewinde in der Spannplatte (18) aufweist.

3. Spannvorrichtung nach Anspruch 2, dadurch gekennzeichnet, dass das Gewindeloch (21) auf der Fixationsseite der Spannplatte (18) angeordnet ist.

4. Spannvorrichtung nach Anspruch 3, dadurch gekennzeichnet, dass ein weiteres Gewindeloch (19) auf der Kompressionsseite der Osteosyntheseplatte (18) zur Fixation der Spannvorrichtung ausgestaltet ist.

5. Spannvorrichtung nach einem der Ansprüche 1 bis 4, dadurch gekennzeichnet, dass der Stift (36) eine Knochenschraube ist.

6. Spannvorrichtung nach Anspruch 5, dadurch gekennzeichnet, dass das Langloch (23) in Längsrichtung beabstandet mehrere paarweise gegenüberliegende Ausbuchtungen (24) zur Aufnahme von Schraubenköpfen aufweist.

7. Spannvorrichtung nach einem der Ansprüche 1 bis 6, dadurch gekennzeichnet, dass der Drehzapfen (33) am plattenfesten Spannelement (11) angebracht ist und mit einem Zahnradgetriebe verbunden ist, dessen Antriebselement auf das bewegliche Spannelement (12) wirkt.

8. Spannvorrichtung nach Anspruch 7, dadurch gekennzeichnet, dass das Antriebselement auf ein mit dem beweglichen Spannelement (12) zusammenwirkendes Schraubenspindelgetriebe (26, 27) wirkt.

9. Spannvorrichtung nach Anspruch 7 oder 8, dadurch gekennzeichnet, dass ein Kegelgetriebe (29, 31) vorgesehen ist.

10. Spannvorrichtung nach einem der Ansprüche 1 bis 6 oder 8, dadurch gekennzeichnet, dass die den Drehzapfen und die Drehspindel (27) verbindende Getriebeanordnung aus einem Kardangelenk besteht.

11. Spannvorrichtung nach Anspruch 9 oder 10, dadurch gekennzeichnet, dass eine Drehspindel (27) drehbar im plattenfesten Spannelement (11) gelagert ist, ein Abschnitt des beweglichen Spannelementes (12) eine mit der Spindel (27) zusammenwirkende Gewindebohrung (26) enthält und das bewegliche Spannelement (12) drehfest, aber verschiebbar am plattenfesten Spannelement (11) gelagert ist.

12. Spannvorrichtung nach Anspruch 11, dadurch gekennzeichnet, dass das bewegliche Spannelement (12) in einer Bohrung (40) des plattenfesten Spannelementes (11) drehfest gelagert ist.

13. Spannvorrichtung nach einem der Ansprüche 1 bis 12, dadurch gekennzeichnet, dass am freien Ende des beweglichen Spannelementes (12) eine Öffnung (35) angeordnet ist, welche den Kopf des Stiftes (36) übergreift.

14. Spannvorrichtung nach Anspruch 13, dadurch gekennzeichnet, dass das freie Ende des beweglichen Spannelementes (12) mit dem Langloch kongruente, zapfenartige Verlängerungen (39) aufweist.

15. Spannvorrichtung nach einem der Ansprüche 1 bis 14, dadurch gekennzeichnet, dass eine Kraftmesseinrichtung im Kraftübertragungsweg zwischen Drehzapfen und beweglichem Spannelement geschaltet ist.

16. Spannvorrichtung nach Anspruch 15, dadurch gekennzeichnet, dass im Kraftübertragungsweg ein fluides, inkompressibles Medium angeordnet ist und die das fluide Medium enthaltende Kammer (103) oder Leitung mit einer Druckmessvorrichtung (115) verbindbar ist.

17. Spannvorrichtung nach Anspruch 16, dadurch gekennzeichnet, dass das bewegbare Spannelement (102) mit einem Kolben (110) verbunden ist, dessen wirksame Fläche einer das fluide Medium enthaltenden Kammer (103) zugekehrt ist und die Getriebeanordnung (107, 108) auf einen weiteren Kolben (105) wirkt, dessen wirksame Fläche ebenfalls der Kammer (103) zugekehrt ist.

**Claims**

1. Clamping device having an osteosynthetic clamping plate provided with screw holes and a slot, also having a force-transmitting device which can be arranged releasably on the clamping plate and which comprises a rotatable stud for introducing a rotational force and co-operates with a pin adapted to extend through the slot, fixable in the bone, and serving as an abutment, characterised in that the force-transmitting device (10) comprises two clamping elements (11, 12) which are movable relatively to one another in the direction of the longitudinal axis of the clamping plate (18) and between which there is arranged a drive arrangement connected to the rotatable stud (33) for producing the relative movement, that the clamping element (11) fast with the plate is fixable non-displaceably to the clamping plate (18) by means of a screw connection, and that the movable clamping element (12) has an abutment surface adapted to be brought into engagement with the head of the pin (36).

2. Clamping device according to claim 1, characterised in that the screw connection for securing the clamping element (11) which is fast with the plate comprises a screw hole (21) with screwthreading in the clamping plate (18).

3. Clamping device according to claim 2, characterised in that the screwthreaded hole (21) is arranged at the fixing side of the clamping plate (18).

4. Clamping device according to claim 3, characterised in that a further screwthreaded hole (19) is provided at the compression side of the osteosynthetic plate (18) for the fixing of the clamping device.

5. Clamping device according to one of claims 1 to 4, characterised in that the pin (36) is a bone screw.

6. Clamping device according to claim 5, characterised in that the slot (23) comprises a plurality of recesses (24) situated opposite one another in pairs and spaced from one another in the longitudinal direction, for receiving screw heads.

7. Clamping device according to one of claims 1 to 6, characterised in that the rotatable stud (33) is arranged on the clamping element (11) which is fast with the plate, and said stud is connected to a toothed-wheel gear whose operating element acts on the movable clamping element (12).

8. Clamping device according to claim 7, characterised in that the operating element acts on a screw spindle transmission (26, 27) co-operating with the movable clamping element (12).

9. Clamping device according to claim 7 or 8, characterised in that a bevel gear (29, 31) is provided.

10. Clamping device according to one of claims 1 to 6 or 8, characterised in that the drive arrangement which connects the rotatable stud and the rotatable spindle (27) consists of a universal-joint connection.

11. Clamping device according to claim 9 or 10, characterised in that a rotatable spindle (27) is mounted rotatably in the clamping element (11) which is fast with the plate, a portion of the movable clamping element (12) includes a screwthreaded bore (26) co-operating with the spindle (27), and the movable clamping element (12) is mounted fast against rotation but displaceably on the clamping element (11) which is fast with the plate.

12. Clamping device according to claim 11, characterised in that the movable clamping element (12) is mounted, fast against rotation, in a bore (40) of the clamping element (11) which is fast with the plate.

13. Clamping device according to one of claims 1 to 12, characterised in that an opening (35) which extends over the head of the pin (36) is arranged on the free end of the movable clamping element (12).

14. Clamping device according to claim 13, characterised in that the free end of the movable clamping element (12) comprises stud-like prolongations (39) congruent with the slot.

15. Clamping device according to one of claims 1 to 14, characterised in that a force-measuring device is interposed in the force transmission path between the rotatable stud and the movable clamping element.

16. Clamping device according to claim 15, characterised in that a fluid incompressible medium is arranged in the force transmission path, and the conduit or chamber (103) containing the fluid medium is connectable with a pressure meter (115).

17. Clamping device according to claim 16, characterised in that the movable clamping element (102) is connected to a piston (110) whose active face is directed towards a chamber (103) containing the fluid medium, and the drive arrangement (107, 108) acts on a further piston (105) whose active face is also directed towards the chamber (103).

**Revendications**

1. Dispositif de tension comportant une plaque de tension ostéosynthétique qui est munie de trous de vis et d'un trou allongé, ainsi qu'un organe de transmission de force pouvant être disposé de façon détachable sur la plaque de serrage, qui présente un tourillon pour l'application d'une force de rotation et coopère avec une broche pouvant être amenée à passer à travers le trou allongé, pouvant être fixée dans l'os et servant de butée, caractérisé par le fait que l'organe de transmission de force (10) est formé de deux éléments de tension (11, 12) pouvant se mouvoir relativement dans la direction de l'axe longitudinal de la plaque de tension (18), entre lesquels est disposé un dispositif de transmission relié au tourillon (33), pour engendrer le mouvement relatif, que l'élément de tension solidaire de la plaque (11) peut être fixé à la plaque de tension (18) sans pouvoir coulisser, au moyen d'un assemblage à vis et que l'élément de tension mobile (12) présente une surface de butée pouvant être amenée en coopération avec la tête de la broche (36).

2. Dispositif de tension selon la revendication 1, caractérisé par le fait que l'assemblage à vis pour la fixation de l'élément de tension solidaire de la plaque (11) présente un trou de vis (21) avec filetage dans la plaque de tension (18).

3. Dispositif de tension selon la revendication 2, caractérisé par le fait que le trou fileté (21) est disposé sur le côté de fixation de la plaque de tension (18).

4. Dispositif de tension selon la revendication 3, caractérisé par le fait qu'un autre trou fileté (19) est aménagé, sur le côté de compression de la plaque d'ostéosynthèse (18), pour la fixation du dispositif de tension.

5. Dispositif de tension selon l'une des revendications 1 à 4, caractérisé par le fait que la broche (36) est une vis pour os.

6. Dispositif de tension selon la revendication 5, caractérisé par le fait que le trou allongé (23) présente plusieurs sinuosités (24) opposées deux à deux, espacées en direction longitudinale, pour loger des têtes de vis.

7. Dispositif de tension selon l'une des revendications 1 à 6, caractérisé par le fait que le tourillon (33) est disposé sur l'élément de tension solidaire de la plaque (11) et est relié à une transmission à roues dentées dont l'élément d'entraînement agit sur l'élément de tension mobile (12).

8. Dispositif de tension selon la revendication 7, caractérisé par le fait que l'élément d'entraînement agit sur une transmission à tige filetée (26, 27) coopérant avec l'élément de tension mobile (12).

9. Dispositif de tension selon l'une des revendications 7 et 8, caractérisé par le fait qu'il est prévu une transmission conique.

10. Dispositif de tension selon l'une des revendications 1 à 6 ou 8, caractérisé par le fait que la disposition de transmission reliant le tourillon et la tige tournante (27) est formée d'un joint à cardan.

11. Dispositif de tension selon l'une des revendications 9 et 10, caractérisé par le fait qu'une tige tournante (27) est montée de manière à pouvoir tourner dans l'élément de tension solidaire de la plaque (11), qu'une partie de l'élément de tension mobile (12) contient un trou fileté (26) coopérant avec la tige (27) et que l'élément de tension mobile (12) est monté, de façon solidaire en rotation mais en pouvant coulisser sur l'élément de tension solidaire de la plaque (11).

12. Dispositif de tension selon la revendication 11, caractérisé par le fait que l'élément de tension mobile (12) est monté de façon solidaire en rotation dans une perforation (40) de l'élément de tension solidaire de la plaque (11).

13. Dispositif de tension selon l'une des revendications 1 à 12, caractérisé par le fait qu'à l'extrémité libre de l'élément de tension mobile (12) est disposée une ouverture (35) qui s'applique par dessus la tête de la broche (36).

14. Dispositif de tension selon la revendication 13, caractérisé par le fait que l'extrémité libre de l'élément de tension mobile (12) présente des prolongements similaires à des tenons (39), de même forme que le trou allongé.

15. Dispositif de tension selon l'une des revendications 1 à 14, caractérisé par le fait qu'un dispositif de mesure de force est branché dans le parcours de transmission de force entre le tourillon et l'élément de tension mobile.

16. Dispositif de tension selon la revendication 15, caractérisé par le fait que dans le parcours de transmission de force est disposé un milieu fluide incompressible et que la chambre (103) ou le conduit contenant le milieu fluide peut être relié à un dispositif de mesure de pression (115).

17. Dispositif de tension selon la revendication 16, caractérisé par le fait que l'élément de tension mobile (102) est relié à un piston (110) dont la surface active est tournée vers une chambre (103) contenant le milieu fluide et que le dispositif de transmission (107, 108) agit sur un autre piston (105) dont la surface active est également tournée vers la chambre (103).

Fig. 1

Fig. 2

Fig. 3